# FASCICULE DE BREVET EUROPEEN

(11) **EP 0 841 328 B1**
(45) Date de publication et mention de la délivrance du brevet: **25.07.2001**
(21) Numéro de dépôt: 97402531.4
(22) Date de dépôt: 24.10.1997
(51) Int. Cl.: C07D 249/08

(54) **Procédé de préparation de 4-amino-1,2,4-triazoles**
Verfahren zur Herstellung von 4-Amino-1,2,4-Triazolen
Process for the preparation of 4-amino-1,2,4-triazoles

(30) Priorité: 07.11.1996 FR 9613590
(43) Date de publication de la demande: 13.05.1998
(73) Titulaire: Atofina, 92800 Puteaux (FR)
(72) Inventeur: Bourdauducq, Paul, 69630 Chaponost (FR)
(74) Mandataire: Neel, Henry

(56) Documents cités:
- EP-A- 0 269 308
- R.M. HERBST: "Studies on the formation of 4-aminotriazole derivatives from acyl hydrazides" JOURNAL OF ORGANIC CHEMISTRY, vol. 18, - juillet 1953 WASHINGTON DC, US, pages 872-7, XP002035182
- CHEMICAL ABSTRACTS, vol. 53, no. 10, 25 mai 1959 Columbus, Ohio, US; abstract no. 9197g, A.N. KOST ET AL.: "Reaction of hydrazine derivatives. XIX. Condensation of 4-amino-1,2,4-triazole with esters" XP002035184 & ZHUR. OBSHCHEI. KHIM., vol. 28, 1958, pages 2773-9,
- DATABASE WPI Section Ch, Week 9610 Derwent Publications Ltd., London, GB; Class A60, AN 96-096242 XP002035185 & RU 2 036 912 C (NOOSFERA-TSENTR RES ENTERP) , 9 juin 1995
- "Catalogue Handbook of Fine Chemicals" 1994 , ALDRICH XP002035183 * page 97, produit A8,180-3 *

## Description

La présente invention concerne un procédé de préparation du 4-amino-1, 2, 4 triazole et plus particulièrement des produits ayant la formule suivante : 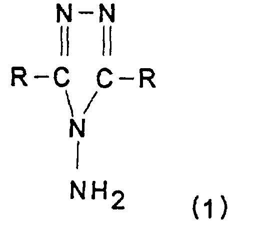 dans laquelle R désigne H ou un groupe alkyle contenant de 1 à 10 atomes de carbone et pouvant être substitué par un ou plusieurs groupes aryles, hétéroaryles, hydroxy ou alcényles. Les deux R peuvent être les mêmes ou différents.

Il consiste à effectuer la réaction de l'hydrazine avec un défaut d'acide carboxylique R COOH compris entre 2 à 5 % par rapport à la stoechiométrie puis à éliminer par distillation l'eau de réaction et éventuellement l'eau de dilution de l'hydrazine et de l'acide.

L'art antérieur US 5 099 028 a décrit un procédé de préparation des mêmes 4-amino-1, 2, 4 triazoles de formule (1) dans lequel on effectue la réaction de l'acide R COOH avec l'hydrazine en présence d'un polymère insoluble contenant des fonctions acides, c'est-à-dire une résine échangeuse d'ions.

S'agissant des produits de formule (1) dans laquelle R est H, c'est-à-dire le 4-amino-1, 2, 4 triazole, l'art antérieur permet d'obtenir après recristallisation dans l'isopropanol un rendement de 85 % et une pureté de 99,5 % si on ne recycle pas la résine ou un rendement de 91 % et une pureté de 99,4 % si on recycle la résine (% en poids).

La demanderesse a découvert qu'il n'était pas nécessaire d'opérer en présence de résines et de plus qu'en utilisant le procédé de l'invention, on diminuait fortement la formation d'une impureté de formule (2) :

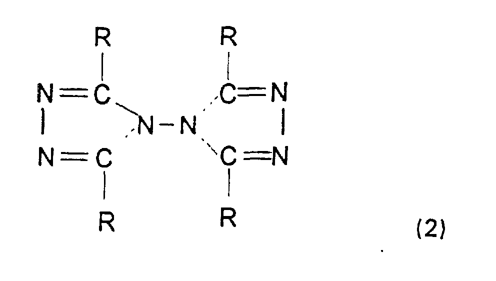

De plus, après recristallisation, on obtient un produit (1) de grande pureté. Par exemple, s'agissant du produit dans lequel R est H la pureté est d'au moins 99,9 % (en poids).

L'invention est particulièrement utile pour préparer le produit de formule (1) dans laquelle R est H, c'est-à-dire le 4-amino-1, 2, 4 triazole.

La présente invention concerne aussi un produit de formule (1) ayant une pureté supérieure à 98,5 % avantageusement supérieure à 99,5 % et de préférence supérieure à 99,9 % (en poids).

L'hydrazine peut être anhydre ou sous forme d'hydrate N₂H₄,H₂O éventuellement en solution aqueuse. Il est suffisant d'utiliser l'hydrate N₂H₄,H₂O en solution aqueuse. Ces solutions peuvent par exemple contenir de 24 à 100 % en poids de N₂H₄,H₂O.

L'acide R COOH peut contenir jusqu'à 20 % d'eau. S'agissant de l'acide formique H COOH, on utilise avantageusement un produit à plus de 90 % de pureté. Le défaut d'acide R COOH peut être de 2 à 5 % et de préférence 2 à 3 % par rapport à la stoechiométrie.

La réaction de l'acide avec l'hydrazine se fait à température ambiante et pression atmosphérique, on laisse monter la température par l'exothermicité de la réaction puis on chauffe vers 130-180° C pour distiller l'eau, avantageusement sous une pression absolue entre 20 et 100 mmHg (26 et 133 h Pa).

Il est avantageux de verser l'acide dans l'hydrazine pour contrôler l'exothermicité de la réaction. On peut aussi commencer à éliminer l'eau dans le début de la réaction. On peut opérer en discontinu ou en continu selon les techniques habituelles de la synthèse organique, ce que l'homme de l'art appréciera.

Selon une forme avantageuse du procédé, le produit (1) est lavé et/ou cristallisé dans un solvant. De préférence, ce solvant est l'isopropanol.

### Exemples

On effectue la synthèse du 4-amino-1, 2, 4 triazole par action de l'acide formique sur l'hydrate d'hydrazine.

Selon la réaction :

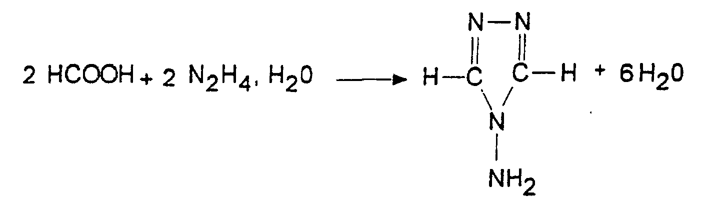

On effectue la réaction avec différentes proportions d'acide formique et d'hydrazine.

On obtient sur les produits bruts avant recristallisation une nette variation de la teneur en impureté (2) (tableau 1).

**Tableau 1**

| Acide formique | Défaut 2,5 % | Défaut 1 % | Stoechiométrie | Excès 5 % |
|---|---|---|---|---|
| Analyse par chromatographie liquide HPLC 4,4'bis 1,2,4-triazole % poids | < 0,1 | 0,3 | 1,3 | 4 |

On décrit ci-après la réaction avec un défaut de 2,5 % d'acide formique suivie d'une recristallisation dans l'isopropanol.

Coulée en 1 h de 29,25 moles d'acide formique provenant d'une solution à 96 % dans 30 moles d'hydrate d'hydrazine provenant d'une solution à 99,5 %.en laissant monter la température à 85° C puis chauffage 7 h en distillant l'eau jusquà 170° C et maintien 2 h à 170° C sous 50 mm Hg.

Refroidir à 80° C et ajouter 1740 g d'isopropanol [4 ATA brut ≅ 42 % poids].

Chauffer à ∼ 75° C jusqu'à dissolution complète et laisser refroidir lentement sous faible agitation.
Filtrer à ≅ 5° C. Laver avec 150 g d'isopropanol froid. Sécher.
Poids de gâteau récupéré = 1186 g
Poids sec = 1008 g
Nous obtenons avec un rendement brut de 82 % un produit très pur.
Analyse par HPLC 99,9 %
Dosage chimique par HClO₄ : 99,7 %
Teneur en eau : 0,14 %

A titre comparatif la même synthèse a été effectuée avec la stoechiométrie en réactifs.

On obtient les résultats suivants (tableau 2) :

**Tableau 2**

| Analyse par chromatographie liquide HPLC % poids | Stoechiométrie | Stoechiométrie puis recristallisation | Défaut d'acide 2,5 % | Défaut d'acide 2,5% puis recristallisation |
|---|---|---|---|---|
| diformylhydrazine | 0,5 | 0,03 | 0,5 | 0,03 |
| monoformylhydrazine | 0,5 | 0,01 | 0,4 | 0,01 |
| 1,2,4-triazole | 0,6 | 0,03 | 0,5 | 0,03 |
| 4,4'bis 1,2,4-triazole | 1,3 | 1,2 | 0,08 | 0,02 |
| 4-amino-1,2,4-triazole | 97,1 | 98,7 | 98,5 | 99,9 |

## Revendications

1. Procédé de préparation de produits de formule (1) : 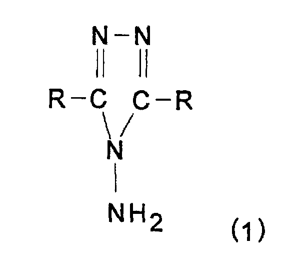 dans laquelle R désigne H ou un groupe alkyle contenant de 1 à 10 atomes de carbone et pouvant être substitué par un ou plusieurs groupes aryles, hétéroaryles, hydroxy ou alcenyles dans lequel on effectue la réaction de l'hydrazine avec un défaut d'acide carboxylique R COOH compris entre 2 à 5 % et de préférence 2 à 3 % par rapport à la stoechiométrie puis on élimine l'eau de réaction et éventuellement l'eau de dilution de l'hydrazine et de l'acide.

2. Procédé selon la revendication 1 dans lequel on effectue ensuite une recristallisation dans l'isopropanol.

3. Procédé selon l'une des revendications précédentes dans lequel R est H.

4. Produit de formule (1) ayant une pureté (en poids) supérieure à 99,5 % et de préférence au moins égale à 99,9 %.

5. Produits selon la revendication 4 dans lesquels R est H.

## Claims

1. Process for the preparation of products of formula (1): 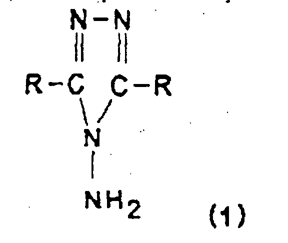 in which R denotes H or an alkyl group containing from 1 to 10 carbon atoms, it being possible for this alkyl group to be substituted by one or more aryl, heteroaryl, hydroxyl or alkenyl groups, in which process hydrazine is reacted with a deficiency of carboxylic acid RCOOH of between 2 and 5% and preferably 2 and 3%, with respect to the stoichiometry, and then the water of reaction and optionally the water of dilution of the hydrazine and of the acid is removed.

2. Process according to Claim 1, in which a recrystallization from isopropanol is subsequently carried out.

3. Process according to either of the preceding claims, in which R is H.

4. Product of formula (1) having a purity (by weight) of greater than 99.5% and preferably at least equal to 99.9%.

5. Product according to Claim 4, in which R is H.

## Patentansprüche

1. Verfahren zur Herstellung von Verbindungen der Formel (1): 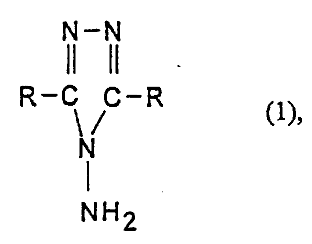 in der bedeuten:
R H oder eine Alkylgruppe mit 1 bis 10 Kohlenstoffatomen, die mit einer oder mehreren Arylgruppen, Heteroarylgruppen, Hydroxygruppen oder Alkenylgruppen substituiert sein kann,
durch Umsetzung von Hydrazin mit einer Carbonsäure RCOOH in einem Unterschuß von 2 bis 5 % und vorzugsweise 2 bis 3 %, bezogen auf die Stöchiometrie, und anschließende Abtrennung des Reaktionswassers und gegebenenfalls des Verdünnungswassers des Hydrazins und der Säure.

2. Verfahren nach Anspruch 1, bei dem anschließend in Isopropanol umkristallisiert wird.

3. Verfahren nach einem der vorhergehenden Ansprüche, wobei R H bedeutet.

4. Verbindung der Formel (1) mit einer (auf das Gewicht bezogenen) Reinheit von mehr als 99,5 % und vorzugsweise mindestens 99,9 %.

5. Verbindungen nach Anspruch 4 mit R = H.
